# EUROPEAN PATENT SPECIFICATION

(11) **EP 1 709 070 B1**
(45) Date of publication and mention of the grant of the patent: **29.05.2013**
(21) Application number: 05701998.6
(22) Date of filing: 24.01.2005
(51) Int. Cl.: C07K 14/62, A61K 39/00

(54) **PEPTIDES FOR TREATMENT OF AUTOIMMUNE DISEASES**
PEPTIDE ZUR BEHANDLUNG VON AUTOIMMUNKRANKHEITEN
PEPTIDES POUR LE TRAITEMENT DE MALADIES AUTO-IMMUNES

(30) Priority: 30.01.2004 GB 0402129; 23.02.2004 US 783095; 25.02.2004 GB 0404199
(43) Date of publication of application: 11.10.2006
(73) Proprietor: KING'S COLLEGE LONDON, London WC2R 2LS (GB)
(72) Inventor: PEAKMAN, Mark, London SE24 9LG (GB)
(74) Representative: Tombling, Adrian George
(86) International application number: PCT/GB2005/000236
(87) International publication number: WO 2005/073248

(56) References cited:
- CONGIA MAURO ET AL: "T cell epitopes of insulin defined in HLA-DR4 transgenic mice are derived from preproinsulin and proinsulin" PROCEEDINGS OF THE NATIONAL ACADEMY OF SCIENCES OF USA, NATIONAL ACADEMY OF SCIENCE. WASHINGTON, US, vol. 95, no. 7, 31 March 1998 (1998-03-31), pages 3833-3838, XP002204284 ISSN: 0027-8424 cited in the application
- GELUK ANNEMIEKE ET AL: "HLA-DR binding analysis of peptides from islet antigens in IDDM" DIABETES, vol. 47, no. 10, October 1998 (1998-10), pages 1594-1601, XP002323217 ISSN: 0012-1797
- NARENDRAN PARTH ET AL: "Humoral and cellular immune responses to proinsulin in adults with newly diagnosed type 1 diabetes." DIABETES-METABOLISM RESEARCH AND REVIEWS, vol. 19, no. 1, 28 October 2002 (2002-10-28), pages 52-59, XP002323390 ISSN: 1520-7552
- LIEBERMAN S M ET AL: "A comprehensive guide to antibody and T-cell responses in type 1 diabetes." TISSUE ANTIGENS, vol. 62, no. 5, November 2003 (2003-11), pages 359-377, XP002323391 ISSN: 0001-2815
- MEIERHOFF GUIDO ET AL: "Cytokine detection by ELISPOT: Relevance for immunological studies in type 1 diabetes." DIABETES-METABOLISM RESEARCH AND REVIEWS, vol. 18, no. 5, October 2002 (2002-10), pages 367-380, XP002323218 ISSN: 1520-7552
- PEAKMAN MARK ET AL: "Naturally processed and presented epitopes of the islet cell autoantigen IA-2 eluted from HLA-DR4" JOURNAL OF CLINICAL INVESTIGATION, vol. 104, no. 10, November 1999 (1999-11), pages 1449-1457, XP002323392 ISSN: 0021-9738 cited in the application
- RAZ I ET AL: "beta-cell function in new-onset type 1 diabetes and immunomodulation with a heat-shock protein peptide (DiaPep277): a randomised, double-blind, phase II trial", THE LANCET, LANCET LIMITED. LONDON, GB, vol. 358, no. 9295, 24 November 2001 (2001-11-24), pages 1749-1753, XP004805546, ISSN: 0140-6736, DOI: 10.1016/S0140-6736(01)06801-5

## Description

This invention relates to autoimmune disease and more particularly to Type 1 diabetes mellitus (T1DM). The objective is the treatment of diabetes and other kinds of autoimmune disease using novel peptide combinations and the use of the same peptide combinations in bioassays designed to monitor this, and other diabetes-specific therapies. The invention will be more specifically described in relation to T1DM, but extension of the novel principles described herein for the therapy of other autoimmune diseases will be apparent from the ensuing description.

In T1DM the immune system inadvertently and progressively destroys the cells in the pancreas that make insulin (beta cells). There is thus a loss of immune tolerance to the beta cell. Eventually there are too few beta cells to ensure proper uptake of blood glucose by body cells and the patient has clinical diabetes. The drawbacks of current treatment for this disease are well known, and research effort has been directed over many years to achieving a greater understanding of the disease process with a view to producing improved methods of early diagnosis and more effective therapies. An effective therapy would be one that restores immunological tolerance to the beta cell. This approach would need to be accompanied by a complementary method for the measurement of beta cell tolerance.

It is well understood that the autoimmune attack on beta cells proceeds by way of the MHC class II pathway, in which antigen presenting cells (APCs) process relevant beta cell protein antigens and present their peptide epitopes to CD4+ T lymphocytes, thereby inducing cytokines which assist in the destruction of the beta cells. One approach which has been proposed in the study of T1DM and other autoimmune disease has been to isolate (elute) the effective epitopes from the complex of peptide and HLA class II molecule and to explore the potential of these peptides for diagnosis and therapy. US patent 5,827,516 is directed to this type of approach for a large number of diseases and US patent 6,562,943 applies this methodology to T1DM. The literature reference corresponding to US 6,562,516 is Peakman et al, 1999, entitled 'Naturally processed and presented epitopes of the islet cell autoantigen IA-2 eluted from HLA-DR4' , J. Clin Invest 104:1449-1457. The term IA-2 indicates Islet Tyrosine Phosphatase-like protein.

Narendan P et al., Diabetes/Metabolism Research and Reviews 2003; 19: 52-59, relate to antibody and T-cell responses against proinsulin and identifies dominant T-cell epitopes.

### Previous attempts to find a solution

These above mentioned disclosures describe the anticipated utility of these isolated peptides in various ways, for example the following:-

### (a) As blocking peptides

This idea was that a peptide, for example, one which binds to the HLA Class II molecule DR4 (hereinafter HLA-DR4), would bind very strongly to this HLA molecule and displace peptides involved in the disease process, thereby preventing activation of autoreactive T cells. This approach was proposed in 1998, before many of the complexities of antigen processing to derive peptides presented by HLA molecules were fully appreciated. This proposal may therefore be difficult to implement, for the following reasons. The manner in which a natural peptide could block activation of T cells is by competitive exchange so that it occupies all possible HLA binding sites and displaces other occupying peptides. Even the highest binding peptide would struggle to compete to block out all other binders. The peptide would have to compete from outside the cell, where no catalytic enzymes are available to help peptide exchange and where the pH (approximately 7.4 extra- as opposed to 5.0 intra-cellularly) is very unfavourable to peptide exchange. It is estimated that this would require at the very least several milligrams of peptide to get a high enough concentration for effective competition. Since the HLA molecules turn over in a matter of minutes/hours on the cell surface, the competitor peptide would have to be constantly available.

It is highly doubtful whether the system could support this hypothesis and we are aware of no published literature to indicate that such peptides and such a therapeutic application and mode of action have been made and used. Furthermore, such a blocking peptide would be globally immune suppressive, rather than specific for T1DM.

### (b) Use of altered peptides

What was contemplated in this proposal was to alter the natural peptide sequence so that it would still bind to the HLA molecule but, instead of activating the T cell, would send a slightly different signal, which would either anergise ("switch off") the T cell, kill the T cell, or switch the T cell to a more benign type. In the 1990s these altered peptide ligands (APL) with antagonistic properties were considered to be highly promising.

The APL approach continues to be pursued in autoimmune disease, although early trials in man have been less encouraging. The problem appears to be that alteration of the natural peptide may render it immunogenic. In two studies in man (in patients with the autoimmune disease multiple sclerosis) the clinical trials were halted because patients developed dangerous allergic immune responses to the APL. These results were reported in the scientific literature in 2000 (refs 1 and 2) below. A further problem was how to identify what alterations would be successful. This proved to be long painstaking work and there was no indication of which peptide to choose, which amino acid to alter and what to change it to.
1. Kappos, L., G. Comi, H. Panitch, J. Oger, J. Antel, P. Conlon, and L. Steinman. 2000. Induction of a non-encephalitogenic type 2 T helper-cell autoimmune response in multiple sclerosis after administration of an altered peptide ligand in a placebo-controlled, randomized phase II trial. The Altered Peptide Ligand in Relapsing MS Study Group. Nat Med 6:1176.
2. Bielekova, B., B. Goodwin, N. Richert, I. Cortese, T. Kondo, G. Afshar, B. Gran, J. Eaton, J. Antel, J. A. Frank, H. F. McFarland, and R. Martin. 2000. Encephalitogenic potential of the myelin basic protein peptide (amino acids 83-99) in multiple sclerosis: results of a phase II clinical trial with an altered peptide ligand. Nat Med 6:1167.

Finally, it is noteworthy that to date no bioassay has been described that is capable of measuring tolerance to beta cells.

### General principles of invention

In accordance, with the present invention we have avoided approaches of the above kind and have focused research effort on ways of using peptides to suppress the specific cells involved in the development of this disease. We have concentrated on key peptides from preproinsulin. We have developed a multi-step approach, incorporating novel steps and bioassays to show for the first time that certain of these peptide epitopes are crucially involved in T1DM development and may be utilised to achieve natural protection from the disease.

The present invention is directed, first, to the problem of how to specifically inactivate the pathogenic CD4+ T lymphocytes responsible for T1DM. This is achieved by (a) identifying the specific peptides recognised by these cells and (b) using them in a therapeutic modality (termed "peptide immunotherapy"). In peptide immunotherapy (PIT) delivery of soluble native peptide leads to the generation/expansion of specialized CD4+ T lymphocytes that are regulatory. Regulatory T cells are capable of specific inhibition of the islet damaging cells by release of anti-inflammatory cytokines, for example interleukin-10 (hereinafter IL-10). Regulatory T cells that operate through release of IL-10 are termed Tr1 cells. Induction of Tr1 cells through PIT is one of the very few therapeutic approaches to offer an outcome in which immunological tolerance to beta cells is restored.

A second problem for which a solution is sought is how to monitor the effect of therapies that are designed to inactivate the CD4+ T lymphocytes responsible for T1DM. Such therapies include PIT, but also other approaches, such as immunosuppressive drugs. This monitoring is achieved by (a) identifying the specific peptides recognised by these CD4+ T lymphocytes and (b) using the peptides in an assay that measures the balance of pathogenic and suppressor CD4+ T lymphocytes through the signature cytokines they make. Such a tolerance assay is critical to the general thrust of preventing T1DM.

Inactivation of pathogenic CD4+ T lymphocytes that recognise specific peptides in the islets is a difficult challenge. Two approaches have been used in the past. The first and most widely used approach attempts to suppress all CD4+ T lymphocytes. Some of these attempts have been successful in showing that therapies aimed at blocking function of CD4+ T lymphocytes can halt progression of diabetes. This is important proof of concept. However, the major problem is that suppressing all CD4+ T lymphocytes leaves the patient open to a very high risk of infection and tumour development as well as the problem of being on the drug long-term with all of the attendant risks that entails. The benefit-to-risk ratio is thus too low for these drugs to be used.

The second approach to inactivating antigen-specific CD4+ T lymphocytes is by administration of the whole antigen, for example by injection or by nasal spray or orally. This approach is thought to lead to the deletion or suppression of pathogenic T cells. There have been attempts with insulin and the published trials have been unsuccessful.

A third way would be to administer specific peptides from the antigen, either as unaltered peptides or as APLs. Peptides have numerous advantages over the use of whole antigen. Peptides are easy to produce, pharmaceutically formulate and quality assure, they do not carry any of the biological side-effects of the parent molecule and weight for weight provide up to 50 times more of the active component (T cell epitope) than whole antigen. However, there are no studies on beta cell peptides as therapeutics in T1DM in man.

We have solved these problems (i.e which peptides to choose for therapy and how to monitor their beneficial effect) in the following ways.

For the problem of choosing which natural peptides to use for therapy, we have extended the approach described in US patent 6,562,943 to load APCs with antigen, to allow their intemalisation, and to identify the peptides that are naturally processed and presented to CD4+ T lymphocytes. In this approach, we have selected preproinsulin as the putative antigen. We have further extended the approach by the inclusion of an algorithm that involves a series of analytical steps, in which natural peptides are selected on the basis of (a) beta cell specificity; (b) affinity for HLA-DR4; (c) recognition by pathogenic CD4+ T lymphocytes, and (d) recognition by regulatory CD4+ T lymphocytes. This approach yields the identity of those peptides most important in the disease and of the greatest potential for immunotherapy. The methodology is described below, including reference to accompanying Tables and Figures.

### Summary of the invention

The invention provides peptides, compositions, methods and uses as specified in the appended claims.

### Description of Figures

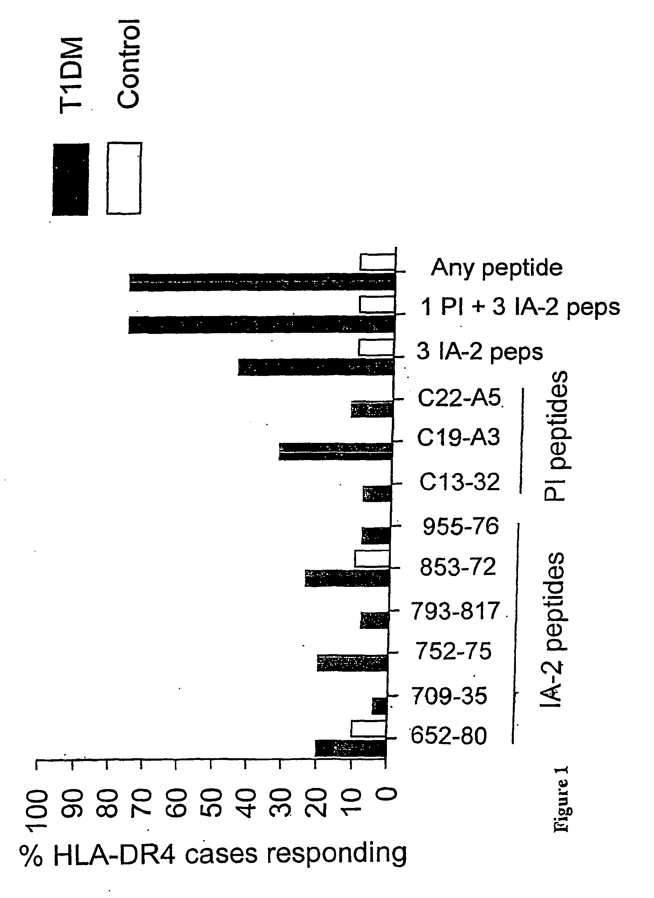

**Figure 1**. Use of IA-2 and preproinsulin peptides to identify pathogenic (IFN-γ) CD4+ T lymphocytes. Graph shows the percentage of HLA-DR4 cases responding amongst type I diabetes mellitus (T1DM) patients (shaded bars) and control non-diabetic subjects (open bars) to each individual IA-2 and preproinsulin (PI) peptides, as well as the response to combinations of peptides from single or multiple antigens. The greatest discrimination between patients and controls using the least peptides occurs when PI C 19-A3 is combined with IA-2 709-36, 752-75 and 853-72 to which 76% of patients and 7% of controls respond (p=0.0001). Patient numbers = 25, controls =14.

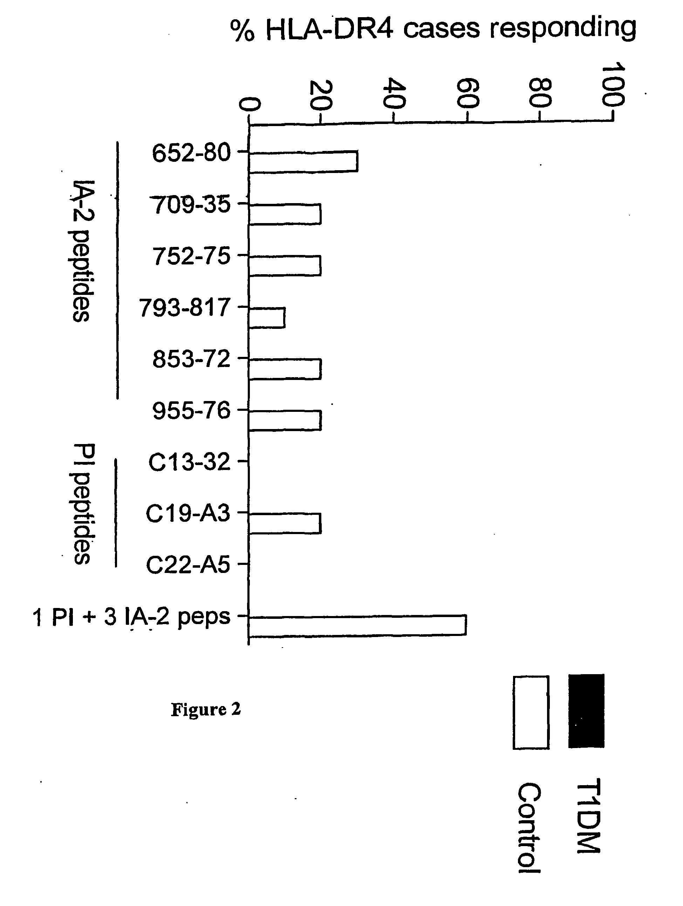

**Figure 2****.** Use of IA-2 and preproinsulin peptides and IL-10 ELISPOT to identify non-pathogenic, anti-inflammatory (IL-10 secreting) (protective) CD4+ T lymphocytes. Graph shows the percentage of HLA-DR4 cases responding amongst T1DM patients (shaded bars) and control non-diabetic subjects (open bars) to each individual IA-2 and preproinsulin (PI) peptides, as well as the response to combinations of peptides from single or multiple antigens by production of IL-10 alone. The greatest discrimination between patients and controls using the least peptides occurs when PI C19-A3 is combined with IA-2 709-36, 752-75 and 853-72 to which 64% of patients and 0% of controls respond (p=0.0001). Patient numbers = 25, controls = 14.

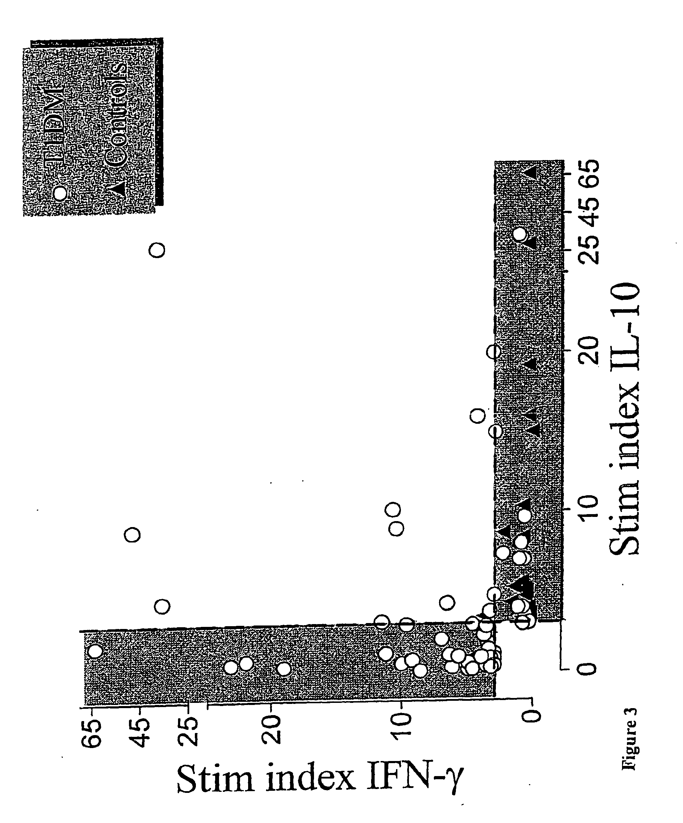

**Figure 3****.** Use of peptides and IFN-γ and IL-10 ELISPOT to identify pathogenic and protective CD4 T lymphocytes in a single assay format Development of an assay that discriminates Type 1 diabetes patients from heathy controls on the basis of their polarization of autoreactive CD4+ T lymphocyte responses to IA-2 and PI peptides. Results of cytokine ELISPOT bioassay is shown for patients with T1DM (open circles) and non-diabetic control subjects (closed triangles). For any given positive peptide response (stimulation index ≥3.0 for IFN-γ or IL-10), the stimulation index for each cytokine has been plotted. There is a highly significant inverse correlation between responses represented by each of these cytokines (p=0.000004), indicating extreme polarization of pro-inflammatory and regulatory autoreactivity. Patients with T1DM are clustered close to the γ-axis, and non-diabetic control subjects distributed along the x-axis, indicating the association of disease and tolerant states with pro-inflammatory and regulatory responses, respectively.

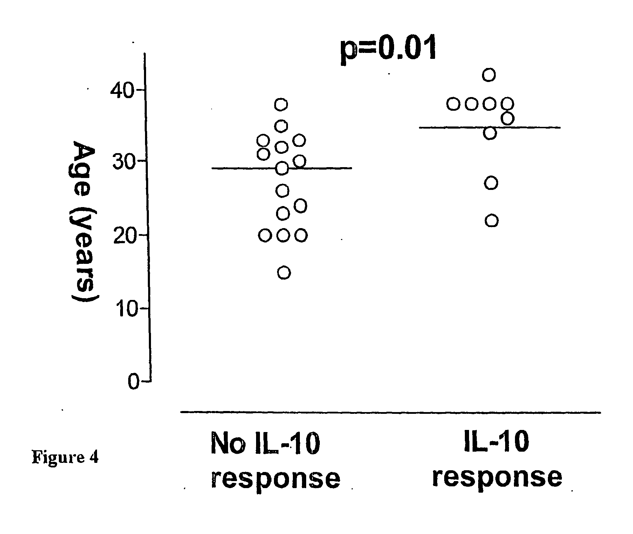

**Figure 4**. The presence of anti-inflammatory (IL-10) CD4+ T lymphocytes delays the onset of diabetes, indicating that these cells have a protective effect through suppression of pathogenic CD4+ T lymphocytes. This is shown by the relationship between age at onset of T1DM and production of IL-10 in response to peptides of IA-2 and preproinsulin. Of patients tested, those making IL-10 responses are significantly older (p=0.01). This provides evidence that anti-inflammatory IL-10 producing CD4+ T lymphocytes that respond to 1A-2 and PI, peptides delay diabetes onset.

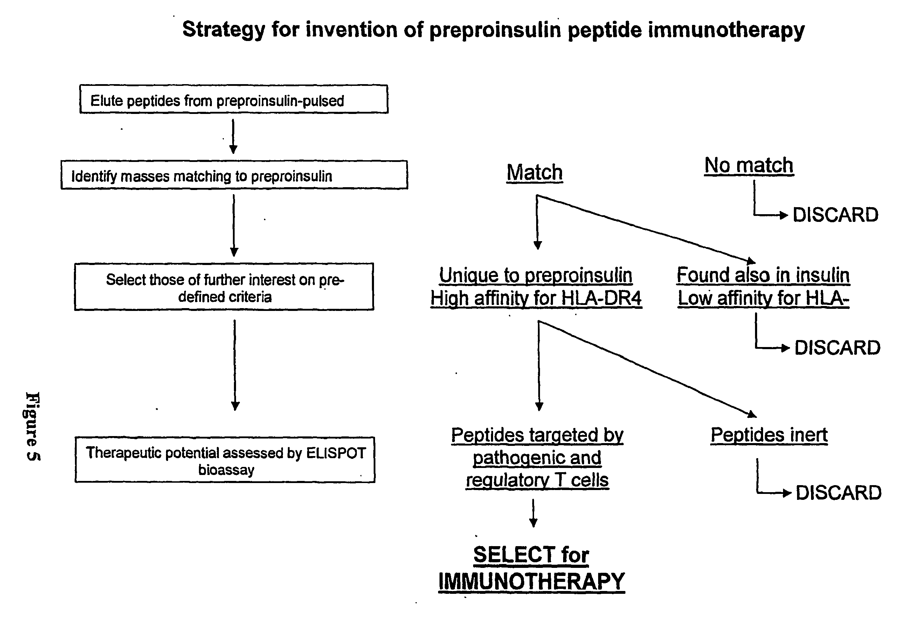

**Figure 5**. Illustration of the sequential multi-step strategy for identification of key preproinsulin peptides for use in peptide immunotherapy.

### METHODOLOGY

The methodology is detailed according to the strategy/algorithm shown in Figure 5.

### 1.1. Identification of peptides of preproinsulin naturally processed and presented by HLA-DR4

cDNA representing the entire sequence of preproinsulin (embl locus HSPPI, accession X70508.1) was cloned into a pET-12a vector (Novagen Inc, Madison WI) modified to include a 6-histidine purification tag and biotinylation sequence at the 5' end and transformed into BLR(DE3)pLysS competent cells (Novagen Inc) for expression and purification under denaturing conditions followed by refolding using a glutathione redox reaction and confirmation of correct folding by analysis of V8 protease digestion products. Recombinant preproinsulin was delivered to the surface of APCs (Priess Epstein Barr virus (EBV) transformed B cells, homozygous for the Type 1 DM-permissive DRB1*0401, [DR4/DRw53], DQA1*0301/DQB1*0302 [DQ8] genotype) and HLA-DR4 purified.

Delivery of preproinsulin to the cell surface at high concentration was achieved using an antigen delivery system (ADS), comprising biotinylated pokeweed mitogen (b-PWM) which binds preferentially to carbohydrate moieties on surface receptors with immunoglobulin-like domains, such as the B cell receptor complex on EBV-transformed B cells, as described in Peakman et al, 1999. Avidin was then used as a bridge between cell surface bound b-PWM and biotinylated preproinsulin. Priess EBV B cells were harvested, washed, counted and resuspended at 10⁸/ml on ice. Cells were then pulsed sequentially on ice for 30 minutes with each component of the ADS at optimal concentrations, comprising b-PWM (Sigma Chemical Co, UK); 1µg/ml), avidin-D (Vector Laboratories; 2mg/ml) and recombinant biotinylated preproinsulin (20µg/ml), with two washing steps with excess cold buffer between each pulse. Pulsed Priess cells were then incubated in RPMI 1640/10% FCS (Life Technologies) at 10⁶/ml for 1 or 6 hours at 37°C, 5% CO₂. Under identical conditions, an equivalent number of Priess cells were pulsed with b-PWM/avidin-D but not biotinylated preproinsulin (control cells). At the end of the incubation period, pulsed Priess cells were washed and stored at -80°C.

HLA-DR4 was purified from Priess cell pellets pulsed with the ADS as follows. Briefly, cell pellets were homogenized in hypotonic buffer and a crude membrane fraction solubilized in 4% NP-40 (Sigma Chemical Co.). The detergent-soluble fraction was passed over a series of immunoaffinity columns made with mAbs specific for HLA class I proteins (W6/32), HLA-DR (L243) coupled to Protein A-Sepharose and HLA-DQ8 (IVD12) coupled to Affigel-10 (Bio-Rad, Hemel Hempstead, UK). The immunoaffinity columns were eluted with 50 mM glycine, pH 11.5, 0.1 % sodium deoxycholate, and immediately neutralized and dialyzed against 10 mM Tris, pH 8.0, 0.1 % sodium deoxycholate. HLA-DR4 was >98% pure as assessed by SDS-PAGE. Immediately before acid-extraction of bound peptides, a 0.5 mg aliquot of HLA protein was passed through an HPLC size exclusion column (ProGel TSK G2000 SW, 7.5 x 300 mm) equilibrated with 10 mM Tris, pH 7.5, to remove molecules not specifically bound to the HLA protein. Fractions containing the HLA-DR4 proteins were concentrated to 100µl by ultrafiltration (Centricon 10, Amicon, MA).

Naturally processed peptide repertoires were acid eluted by incubation for 15 minutes at 70°C with 800 µl 10 % acetic acid and isolated from the remaining HLA protein by ultrafiltration through the Centricon 10. Acid-extracted peptides were vacuum concentrated to approximately 20-30 µl and separated by RP-HPLC, using a microbore C₁₈ column (1.0 x 250 mm; Vydac, Hesperia, CA) at 50-200 µl/minute. Samples were air-dried and approximately 2 % loaded onto a sample plate along with 0.4 µl of matrix (α-cyano-4-hydroxycinnamic acid, 10 mg/ml in 50 % acetonitrile/0.1 % trifluoroacetic acid) and allowed to air dry. Mass spectra were collected at optimum laser intensities by averaging the ion signals from 256 individual scans in both linear and reflector modes using a single stage extended length reflector time-of-flight mass spectrometer (Voyager Elite XL; PerSeptive Biosystems, Framingham, MA). Time to mass conversion was performed by external calibration using synthetic peptides. Mass accuracy varied from 0.03% to better than 0.01% in linear and reflector modes, respectively.

The mass spectra for the HLA-DR4 peptide repertoire isolated from Priess cells pulsed with preproinsulin and the control preparation were compared to identify novel m/z values corresponding to peptides derived from preproinsulin. Five masses were identified as being unique to the preproinsulin- pulsed peptide preparation, corresponding to seven preproinsulin sequences (see Table 1). All sequences span an extended region of preproinsulin from the end of the B chain to the middle of the A chain. The peptides circumscribed two potential nested sets that are characteristic of class II MHC processing (C3-C27 and C13-A5).

A second selection step for preproinsulin peptides of therapeutic interest was then applied, using the following strategy. The immune attack in Type 1 diabetes is directed against the β cell alone. Preproinsulin is unique to β cells. In contrast, by virtue of the fact that it is a secreted hormone required for physiological activity throughout the body, insulin (and its discarded, secreted connecting chain, the C-peptide) has a ubiquitous distribution. Eluted peptides were therefore categorised for their presence in preproinsulin alone, versus presence in preproinsulin and insulin/C-peptide. Five of the sequences fulfilled the criterion (SEQ ID NO: 1 and 4-7) of being present in preproinsulin alone.

A further selection step was applied based on the following novel initiative. Only those preproinsulin peptide sequences with high measurable affinity for HLA-DR4 would have peptide immunotherapeutic potential. Synthetic preproinsulin peptides based on the sequences of the five candidates were therefore assessed for their ability to bind soluble HLA-DR4 in vitro in a direct competition binding assay against a biotinylated indicator peptide (98-117 of the MHC class II invariant chain) as follows. HLA-DR4 molecules used in this assay were purified from resting, unmanipulated Priess cell pellets, using the immunoaffinity column approach described above in method 1.1. To perform the binding assay, 12µg of immunoaffinity-purified HLA-DR4 were incubated with 2.5µM biotinylated indicator peptide and test peptide or non-biotinylated indicator peptide at a range of concentrations (0.1-100µM) in a final DMSO concentration of 20% in 20mM 2-[N-morpholino]ethanesulfonic acid, 1% w/v n-octylglucoside, 140mM sodium chloride, 0.05% sodium azide, pH 5, for 20 hours at room temperature. Peptide mixtures were transferred to wells of a Maxisorp plate (Nalge Nunc, Hereford, UK) that had been pre-coated for 20 hours at room temperature with 100µl of anti-HLA-DR (L243) capture antibody at 10µg/ml in phosphate buffered saline (PBS), blocked with 3% non-fat dried milk and 3% bovine serum albumin (BSA) for 30 minutes each and washed 5 times in Tris buffered saline (TBS)/0.1% Tween-20 (all chemicals from Sigma Chemical Company, Poole, Dorset). Plates were incubated for a further 1 hour at room temperature and washed 5 times in TBST. Europium-conjugated streptavidin (Perkin Elmer Ltd., Hounslow, UK) was added at 1µg/ml in dissociation-enhanced time-resolved fluoroimmunoassays (DELFIA) assay buffer (Wallac Oy, Turku, Finland) and incubated for 45 minutes at room temperature. Wells were washed a further 5 times in TBST and 100µl DELFIA enhancement solution added to each well. Fluorescent intensity was measured in a DELFIA fluorimeter.

Binding affinity was expressed as an inhibitory concentration 50 (IC₅₀), determined as that required to inhibit binding of 2.5µM biotinylated indicator peptide by 50%. Only those peptides with high affinity for HLA-DR4 (IC₅₀ <10 µM) were selected. One of the nested sets of peptides (C13-A5), containing 3 peptide sequences (SEQ ID NO:4-6) fulfilled these criteria.
The results of this step-wise analysis are shown in Table 1.

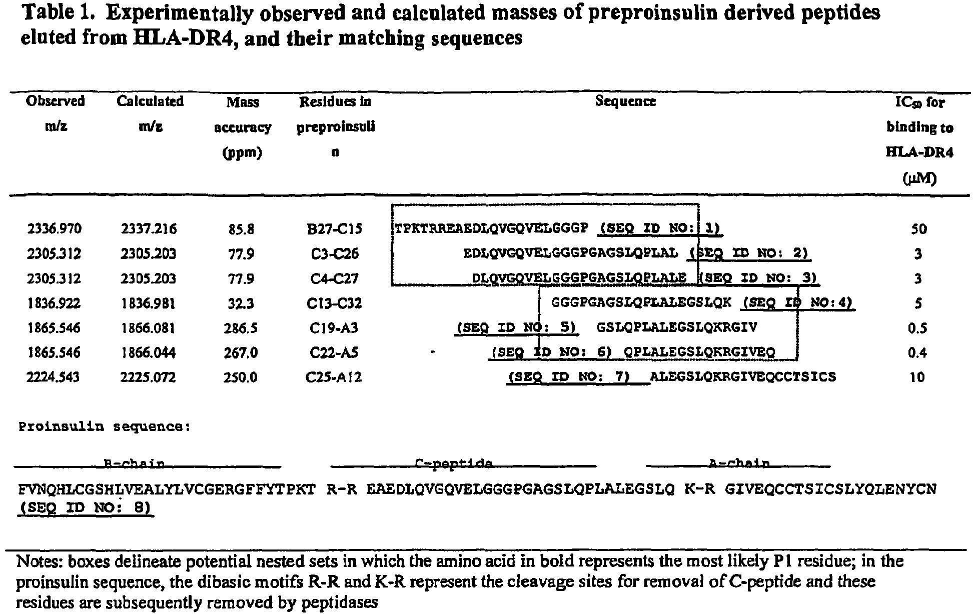

The peptides SEQ ID Nos 1 to 7 shown in Table 1 above are those presented by DR4 and derived by cellular processing. It will be understood that these do not constitute epitopes (ie no evidence is provided from elution alone that these peptides are recognised by CD4 T cells) and therefore have no disease relevance taken alone. It cannot be concluded from these data alone that any of these peptides have therapeutic utility for the purposes of the present invention.

### 1.2 Second phase of identification of peptides of therapeutic potential by analysis of patient responses to candidate peptides using the IFN-γ ELISPOT.

Our initial screening approach determines which peptides are naturally presented, which have excellent binding characteristics to HLA-DR4, and in the case of preproinsulin, which sequences are unique to this molecule and absent in mature insulin, but not which ones the pathogenic CD4+ T lymphocytes react against during the immune response that leads to T1DM. To solve this problem, we have taken the candidate peptides from preproinsulin and others identified from IA-2 disclosed in Peakman et al 1999 and tested each of these individually in an assay format called a cytokine ELISPOT. This detects the signature of a CD4+ T cell according to the cytokine it makes. Making interferon-γ (IFN-γ) represents a pathogenic CD4+ T lymphocyte response. The important peptides from a disease point of view are those that elicit a pathogenic response in this assay. This is therefore a very critical refinement of the simple approach to epitope identification above because it reveals which epitopes are important in the disease context. Prior to the present invention, this approach has not been previously disclosed or carried out with IA-2 peptides or any other peptides.

### INTERFERON-γ ELISPOT ASSAY PROCEDURE

Fresh heparinised blood was obtained from 25 Caucasian Type 1 DM patients with HLA-DR4 and acute onset of symptoms, requiring insulin from diagnosis, and from 14 non-diabetic healthy control subjects matched for age and HLA type. Peripheral blood mononuclear cells (PBMCs) were isolated fresh on density gradient (Lymphoprep, Nycom Pharma, Norway) and washed in RPMI 1640 (Life Technologies, Paisley, UK) twice before use. Fresh PBMCs in RPMI 1640 supplemented with antibiotics (TC medium; all Life Technologies) and 10% human AB serum (Harlan SeraLab, Leicestershire, UK) were dispensed into 48-well plates at a density of 2x10⁶ in 0.5ml supplemented with the selected peptide to a final concentration of 10µM and incubated at 37°C, 5% CO₂, tilted by 5°. Control wells comprised TC medium containing an equivalent concentration of peptide diluent alone (DMSO), tetanus toxoid (final concentration 100ng/ml), or PMA/ionomycin (5ng/ml and 745 ng/ml final concentrations, respectively).

On day +1, 0.5ml pre-warmed TC medium/10%AB was added and on day +2, non-adherent cells were re-suspended using pre-warmed TC medium/2% AB, washed, brought to a concentration of 1x10⁶/300µl and 100µl dispensed in triplicate into wells of 96-well ELISA plates (Nunc Maxisorp, Merck, Poole, UK) pre-blocked with 1% BSA in PBS and pre-coated with monoclonal anti-IFN-γ capture antibody (U-Cytech, Utrecht, NL). After capture at 37°C, 5% CO₂ for 7 hours, cells were lysed in ice cold water, plates washed in PBS/Tween 20 and spots developed using anti-IFN-γ detection antibody and an appropriate revealing agent. Plates were dried and spots of 80-120µm counted in a BioReader 3000 (BioSys, Karben, Germany). Mean values in test wells were compared with means of the background (DMSO) wells to derive a stimulation index (SI).

The results are summarised in Figure 1 and Table 3. Figure 1 shows the percentage of diabetic patients and controls responding by production of IFN-γ to one or other of the 6 IA-2 peptides and 3 preproinsulin peptides. The results demonstrate that each of the three preproinsulin peptides tested elicits the type of immune response associated with a pathogenic T cell. It is evident that responses are more prevalent in patients and that the greatest discriminative power (between patients and controls) is seen when a minimum of 1 preproinsulin peptide (C19-A3) and 3 IA-2 peptides (709-736, 752-775 and 853-872) are used. In combination, these particular peptides thus represent a cocktail that has the highest achievable disease relevance.

Amongst the 25 patients tested against both IA-2 and PI peptide panels, an IFN-γ response to at least one peptide was seen in 18/25 (72%) T1DM patients, compared with 1/14 (7%) non-diabetic control subjects (p=0.0001). This increase in diagnostic sensitivity was not achieved at the loss of specificity, since none of the non-diabetic control subjects made IFN-γ responses to any of the PI peptides.
Overall, responses to the IA-2 and PI peptides, which had been identified by elution from HLA-DR4, tended to be higher in patients with at least one HLA-DR4-encoding allele. Thus, 15/25 (60%) and 10/17 (59%) patients with at least one HLA-DR4 molecule responded to at least one IA-2 or PI peptide respectively, compared with 4/11 (36%) and 4/8 (50%) of patients with non-DR4 alleles, Similarly, the prevalence of responses to either peptide panel was greater amongst those patients with at least one HLA-DR4 allele (13/17, 76%) compared with those with no -DR4 alleles (5/8, 63%) although none of these trends were significant with the numbers of cases tested in this study.
Additional studies were carried out using samples from 4 T1DM subjects with islet peptide reactive T cells to examine the nature of the responding cells. Positive responses (SI≥3.0) were entirely abolished when PBMCs were depleted of CD4 T cells, indicating that the autoreactive T cells detected are CD4+. In addition, we were able to examine the persistence of IFN-γ T cell responses in a further 4 T1DM patients (all DRB1*0401) from whom a second blood sample was available 15-23 weeks after the first. In three patients there was a positive IFN-γ T cell response (SI ≥3.0) in the first sample to at least one IA-2 peptide. In two of these patients, the positive responses remained, whilst in the third, the response to one peptide persisted and to the other declined. The fourth patient showed no response in either sample. These results indicate that, when present, pro-inflammatory autoreactive T cell responses have a tendency to persist during the first months after diagnosis.

Summarising these results, there is a clear association between detectable pathogenic IFN-γ responses to selected preproinsulin and IA-2 peptides and the diagnosis of Type 1 diabetes.

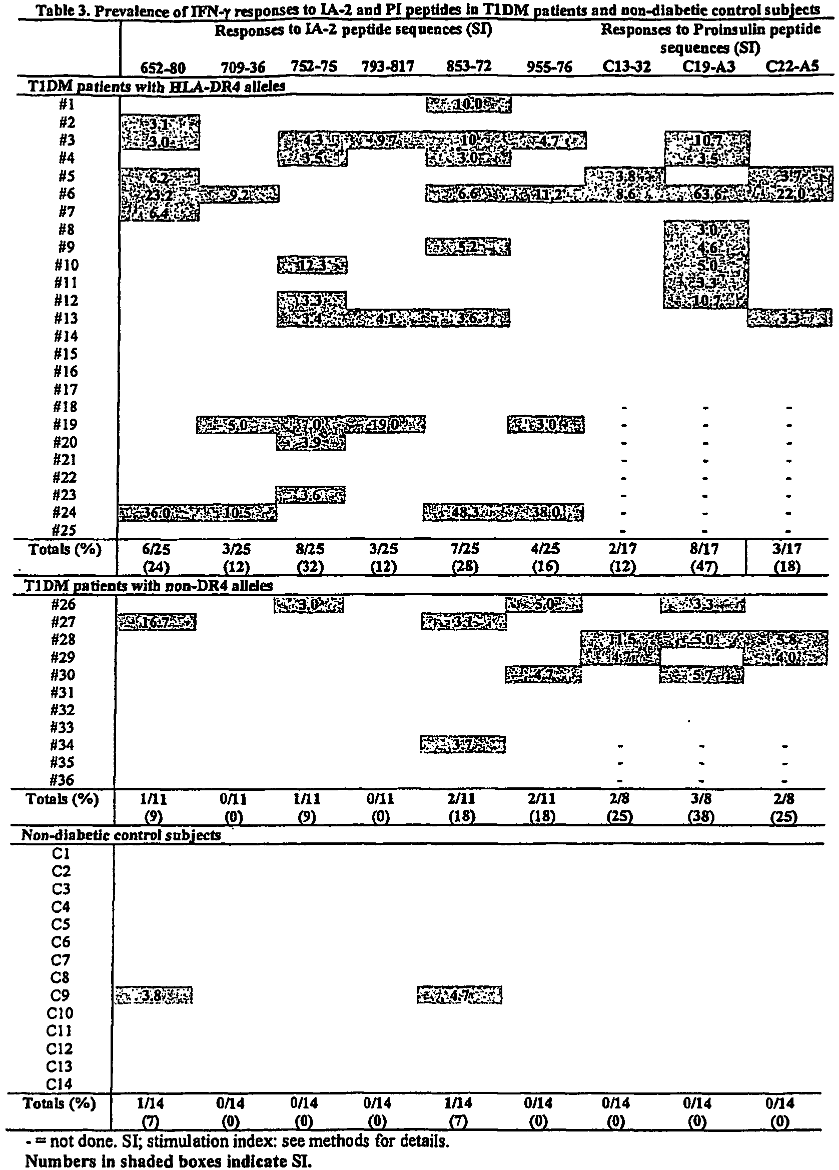

### 1.3 Third phase of identification of preproinsulin peptides of therapeutic potential by analysis of patient responses to candidate peptides using the IL-10 ELISPOT.

In the final step of peptide selection, we sought to identify those peptides with potential efficacy in the manipulation termed "peptide immunotherapy". As stated previously, this therapeutic approach exerts its effect through the induction/recruitment of specialized regulatory CD4+ T cells (Tr1 cells) that produce IL-10, and suppress active inflammation in autoimmune disease. Such cells can be detected using the IL-10 ELISPOT. We searched for IL-10 producing, peptide specific Tr1 cells in a group of patients with T1DM, some of whom had early onset of disease and some of whom had late onset of disease.

Our aim was to identify peptides recognised by Tr1 cells in patients with late onset disease. Disease which appears at a later age is likely to reflect slow beta cell destruction, associated with attempts by the immune system to regulate pathogenic T cells (e.g induction of regulatory T cells). We sought to identify peptides recognised by regulatory T cells. The procedure used is identical to that in section 1.2, apart from the following differences:
On day +1, 0.5ml pre-warmed TC medium/10%AB was added and on day +2, non-adherent cells were re-suspended using pre-warmed TC medium/2% AB, washed, brought to a concentration of 1x10⁶/300µl and 100µl dispensed in triplicate into wells of 96-well ELISA plates (Nunc Maxisorp, Merck, Poole, UK) pre-blocked with 1% BSA in PBS and pre-coated with anti-IFN-γ or monoclonal anti-IL-10 capture antibody (U-Cytech, Utrecht, NL). After capture at 37°C, 5% CO₂ for 7 hours, cells were lysed in ice cold water, plates washed in PBS/Tween 20 and spots developed using either anti-IFN-γ or anti-IL-10 detection antibody and an appropriate revealing agent. Plates were dried and spots of 80-120µm counted in a BioReader 3000 (BioSys, Karben, Germany). Mean values in test wells were compared with means of the background (DMSO) wells to derive a stimulation index (SI).

Results are shown in Table 4 and Figures 2 and 4. Figure 2 shows the percentage of diabetic patients and controls responding by production of IL-10 alone to one or other of the 6 IA-2 peptides and 3 preproinsulin peptides. The results demonstrate that each of the three preproinsulin peptides tested elicits the type of immune response associated with a regulatory T cell. It is also evident that responses are more prevalent in non-diabetic patients and that the greatest discriminative power (between patients and controls) is seen when the minimum of 1 preproinsulin peptide (C19-A3) and 3 IA-2 peptides (709-736, 752-775 and 853-872) are used. In combination these peptides thus represent the most relevant to identifying the protective phenotype.

A striking finding was that more than half of the non-diabetic control subjects (9/14, 64%) made IL-10 responses to IA-2 peptides, compared with a minority of patients with newly-diagnosed T1DM (7/24, 29%; p<0.05, Table 4). These responses were frequently directed against multiple epitopes and of considerable magnitude. Repeated testing one month later in 4 of the non-diabetic control subjects showed that the IL-10 response was reproducible over time (ie 4/4 subjects showed responses classed as positive, SI≥3.0 to the same peptides as in the original assay).

Summarising these data on IL-10 responses, there is a clear trend for an IL-10 response against IA-2 peptides to discriminate patients and control subjects (p<0.05). This trend remains for combined anti-IA-2 and anti-preproinsulin responses (p=0.08) when only the HLA-DR4 cases and controls are considered (consistent with DR4-eluted peptides being more discriminatory amongst DR4 subjects). IL-10 responses to preproinsulin appear non-discriminatory, although fewer cases were studied.

We made the further novel discovery that patients with T1DM who made IL-10 responses to either IA-2 or preproinsulin peptides tended to be significantly older at diagnosis of disease (by an average of 7.5 years) than those who did not (p=0.01; Figure 4), thus suggesting that this quality of response is associated with a later disease onset, indicating a protective effect of IL-10 production.

In summary, we identified for the first time a series of peptides that are the targets of naturally arising Tr1 cells. The Tr1 cells are clearly associated with two conditions. The first is the healthy, non-diabetic state. The second is the late or slow onset of disease. These results firmly link these Tr1 cells with protection from diabetes development. In this respect, the peptides that are targets of Tr1 cells show ideal properties for use in a peptide immunotherapeutic setting.

In accordance with the present invention, therefore, the use of the novel series of selection steps described above and illustrated in outline in Figure 5 has enabled us to determine the sequences of crucial peptides and peptide combinations effective for the therapeutic or prophylactic control of T1DM. Three such peptides are those having the following sequences :-
GGGPGAGSLQPLALEGSLQK (SEQ ID NO: 4),
GSLQPLALEGSLQKRGIV (SEQ ID NO: 5),
and QPLALEGSLQKRGIVEQ (SEQ ID NO: 6)
of which that having the sequence GSLQPLALEGSLQKRGIV (SEQ ID NO: 5) is highly preferred. Also comprised within the present invention is a peptide which contains the following consensus of the above three sequences :-
GGGPGAGSLQPLALEGSLQKRGIVEQ (SEQ ID NO: 10).

It is apparent that an essential component sequence of the above peptides is.the sequence :-
QPLALEGSLQK (SEQ ID NO: 9).
which sequence is extended at one or both ends thereof in the peptides defined above. The present invention therefore comprises a peptide having a sequence comprising or consisting of QPLALEGSLQK (SEQ ID NO: 9).

As indicated in Table 1, the peptides for use according to the present invention are components of the preproinsulin molecule. No therapeutic activity has previously been ascribed to these novel peptides separated from sequences with which they are associated in preproinsulin. Similarly, no therapeutic activity has been hitherto discovered for peptides having other subsequences of the preproinsulin molecule for which immunogenic properties have been disclosed, including sequences described by Congia et al based solely on experiments in mice (Proc. Natl. Acad. Sci. U.S.A. vol 95 :3833-3838). In particular, it should be noted that there is no agreement among those skilled in the art as to what peptide components of preproinsulin are immunodominant peptides. In these circumstances, there has been no clear prior indication or suggestion of therapeutic activity associated with peptides having such previously disclosed subsequences. In contrast, the peptides of the present invention have been demonstrated as having therapeutic or preventive activity especially in relation to T1DM. These peptides are especially useful for the treatment of patients that have the HLA-DR4 allele.

Distinction of the inventive peptides from those having the larger sequences as occurring in Nature may be expressed by the term "isolated or purified " peptides (e.g in the senses used in US 6,562,943 B1), although it will be understood that for practical use these peptides will preferably be synthesised and produced to specification in accordance with regulatory requirements. Peptides within the scope of the present invention may also be described in general terms as peptides consisting essentially of at least one of the sequences SEQ ID Nos 4,5,6,9 and 10.

Other possible extensions of the sequence SEQ ID NO :9 are also comprised within the scope of the present invention, along with minor extensions of the peptides SEQ ID Nos 4,5,6,and 10. Extended peptides of this kind which retain the therapeutic potential of the peptides SEQ ID Nos 4,5,6, 9, and 10 may be readily selected by application of selection criteria 1.2 and 1.3 described above.

For use in the therapeutic or prophylactic treatment of T1DM, at least one of the above peptides will be the primary component of the pharmaceutical composition supplied for such use. Various combinations of two or more of these peptides may be used if desired.

The present invention also comprises one or more of the above sequences in combination with one or more peptides from IA-2 that have now been found to exhibit good synergy with preproinsulin peptide C19-A3 (SEQ ID NO 5). These are shown in Table 2 below.

**Table 2. IA-2 peptides eluted from HLA-DR4 that have synergy with preproinsulin 75-92**

| Numbering in IA-2 | Sequence |
|---|---|
| 709-36 | LAKEWQALCAYQAEPNTCATAQGEGNIK (SEQ ID NO: 11) |
| 752-75 | KLKVESSPSRSDYINASPIIEHDP (SEQ ID NO: 12) |
| 853-72 | SFYLKNVQTQETRTLTQFHF (SEQ ID NO: 13) |

It will be appreciated from the foregoing description that the selection of the above peptides or peptide combinations has resulted from methodology which is also novel and which may be used to determine other possible peptides associated with T1DM and combinations which will prove effective in the treatment or control of T1DM. For example, the novel methodology is applicable to the selection of peptides or peptide combinations comprising preproinsulin-derived and IA-2- derived peptides eluted from HLA-DR3, HLA-DQ8 and HLA-DQ2 and Glutamic acid decarboxylase (GAD)65 eluted from HLA-DR4, HLA-DR3, HLA-DQ8 and HLA-DQ2.

Indeed this methodology is of such broad applicability that it permits extension to the search for and selection of therapeutically valuable peptides or peptide combinations derived from autoantigens linked to other autoimmune diseases besides T1DM. Thus, as applied in the T1DM example described in detail herein, the method will begin with elution of peptides held within the corresponding MHC Class II complex and will proceed with selection steps and criteria corresponding to those described herein (1.1, 1.2, and 1.3 above). Examples of such other autoantigens and their diseases to which this methodology is applicable include:
1. In relation to multiple sclerosis, the elution of peptides derived from (i) myelin basic protein; or (ii) myelin oligodendrocyte glycoprotein; or (iii) proteolipid protein from HLA-DR2;
2. In relation to rheumatoid arthritis, the elution of peptides derived from (i) collagen; or (ii) binding immunoglobulin protein (BIP); or (iii) citrullinated filaggrin from HLA-DR4.

In general terms, therefore, the present invention provides a method of assessing the potential of a peptide for use in the therapy or prevention of an autoimmune disease, which comprises subjecting the candidate peptide to a first assay indicative of a pathogenic T cell response in blood (or other biological sample) (e.g an ELISPOT for IFN-γ) and optionally, in the case of a positive response thereto, subjecting the candidate peptide to a second assay indicative of a regulatory T cell response to the peptide (eg an ELISPOT for IL-10). Where both such assays are used, the peptide giving a positive response in the second assay will be selected for therapy.

### 2. Tolerance assay: Development of an assay to measure immunological tolerance to beta cells.

Many/most of the at-risk individuals who may be treated to prevent future diabetes have no symptoms. They are identified as being at risk by a blood test for autoantibodies and genes, either as part of a population-wide screening programme, or because they have a close relative with diabetes. If they have no symptoms or signs, one cannot know whether the therapy is having an effect, without having to wait 5-10 years to see whether they develop diabetes or not. In other words, the whole field of intervention therapy in diabetes needs surrogate markers of therapeutic efficacy ("tolerance assays"). Having identified appropriate peptides for use as described above, we have now developed a bioassay that measures tolerance; the balance of pathogenic and suppressor CD4+ T lymphocytes. No such assay has been available hitherto.

The procedures used are those described in sections 1.2 and 1.3 above, to perform a cytokine ELISPOT on peripheral blood for the combined detection of IFN-γ and IL-10.

The results of the combined assay analysis on fresh heparinised blood obtained from 25 Caucasian Type 1 DM patients with HLA-DR4 and acute onset of symptoms, requiring insulin from diagnosis, and from 14 non-diabetic healthy control subjects matched for age and HLA type are plotted in Figure 3. To examine the nature of the relationship between IL-10 and IFN-γ responses to IA-2 and preproinsulin peptides in patients and control subjects, we plotted the stimulation index for each cytokine when a positive peptide response was observed (SI≥3.0 for IFN-γ or IL-10). These results demonstrated a highly significant inverse correlation between responses represented by each of these cytokines (Figure 3; p=0.000004), indicating that in the context of an autoreactive T cell response there is extreme polarization of pro-inflammatory *versus* regulatory autoreactivity. Moreover, whilst patients with T1DM were clustered close to the y-axis, non-diabetic control subjects were distributed along the x-axis, highlighting the association of the disease and tolerant states with pro-inflammatory and anti-inflammatory or regulatory responses, respectively. In contrast, there was no inverse correlation between IFN-γ and IL-10 responses to tetanus toxoid (p=0.64).

This tendency to make either polarized Th1 or regulatory T cell responses to naturally processed and presented IA-2 and preproinsulin epitopes provides a clear distinction in the quality of autoreactivity between T1DM patients and non-diabetic subjects (p<0.0001).
Thus the tolerance assay we describe, when plotted as in Figure 3, indicates that the combination of the selected peptides and an assay that measures IFN-γ and IL-10 responses can discriminate patients and control subjects into 3 broad categories. Category 1, along the x-axis (IL-10 but no IFN-γ), is the healthy non-diabetic state. Category 2, along the y-axis (IFN-γ but no IL-10), is the disease state. Category 3, in the upper right quadrant (IFN-γ and IL-10) is the slowly progressive disease state. This novel approach identifies responses in categories 1 and 3 as representing measurable degrees of tolerance. Patients with new-onset or pre-diabetes, undergoing immunotherapy and in whom tolerance is being measured using this assay, will be predicted to make a shift from category 2, to the right and down (reader is referred to Figure 3), as an indication of tolerance induction and treatment effect.

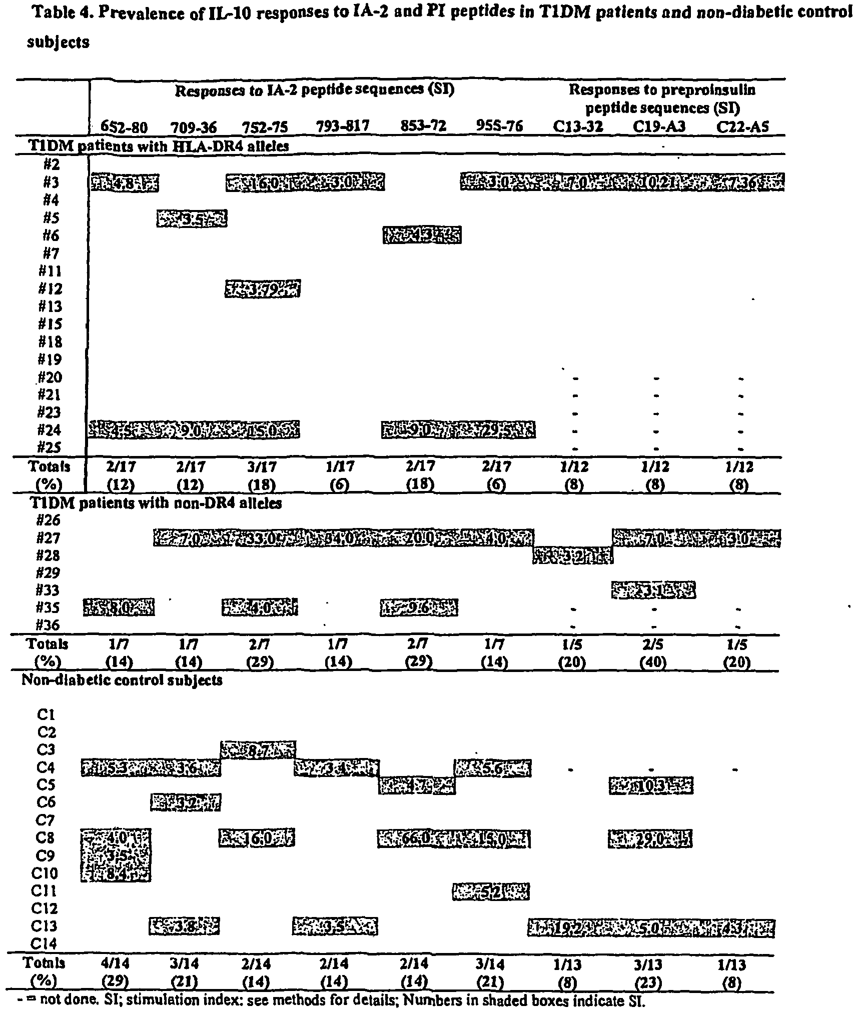

### (1) Specific therapy for Type 1 diabetes.

We have made a discovery that provides strong support for the pursuit of this novel strategy. When analysing responses of PBMCs from patients with Type 1 diabetes to the preproinsulin and IA-2 peptides, we identified these peptides as the targets of a regulatory T cell subset These Tr1 cells were found in patients with slowly progressive disease. This provides in vivo evidence that Tr1 cells recognising the preproinsulin and IA-2 peptides have regulatory and tolerogenic properties. Since Tr1 cells recognizing these peptides may be induced by peptide immunotherapy, we propose the therapeutic use of preproinsulin and IA-2 peptides in diabetes prevention.

Our strategy for peptide immunotherapy is the use of peptide injection, or related methods, to induce in vivo regulatory populations of T cells (Tr1 cells) that recognise the same peptide as the equivalent effector pathogenic T cells (these cells make IFN-γ and are called Th1). Peptide immunotherapy is particularly potent at inducing Tr1 cells that synthesise the immunosuppressive chemical mediator IL-10. Under these circumstances, when one of the preproinsulin and IA-2 peptides is presented by an antigen presenting cell in the pancreas or local lymph nodes in a patient developing diabetes, the peptide is recognised simultaneously by Th1 and Tr1 cells. It is known that under these circumstances the Tr1 cell is dominant and exercises "bystander suppression" over the Th1 response.

The identification of a specific combination of peptides that identifies pathogenic CD4+ T lymphocytes in a majority of patients leads us to a therapy by which CD4+ T lymphocytes involved in T1DM are inactivated, restoring long-term beta cell tolerance. An important element in this approach is the demonstration that the combination of 4 epitopes from 2 autoantigens is outstanding in terms of its coverage of pathogenic CD4+ T lymphocyte responses. Such coverage gives a greater potency to the therapy, and applicability to a wider range of patients, than any mono-therapy hitherto proposed. Thus our therapeutic approach is multi-epitope, multi-antigen peptide immunotherapy and uses the peptides we have identified through a combination of elution and bioassay.

An example of an aspect of the invention is as follows. The selected peptides are synthesized to GMP grade and pooled in order to represent the best possible combined efficacies. Peptides are used singly or pooled in vials containing up to about 1 mg of each peptide per single dose e.g from 0.5 to 5 to 50 to 250 or up to 500 µg in sterile saline and the vial contents administered. In general, and as used in this example, administration can be by parenteral or oral or topical routes including intradermal, subcutaneous or intravenous injection, or nasally or orally or epicutaneously as simple solutions. Peptides may also be given in conjunction with tolerance-promoting adjuvants or tolerance promoting cells. Tolerance promoting adjuvants include IL-10 and recombinant cholera toxin B-subunit (rCTB), which are co-administered with peptide. Tolerance promoting cells include immature dendritic cells and dendritic cells treated with vitamin D3, (1alpha,25-dihydroxyvitamin D3) or its analogues. In this example, immature dendritic cells are expanded from patient blood in vitro using standard techniques before the commencement of therapy. Peptides are then bound to the dendritic cells in vitro before administration, which may be by any of the parenteral routes mentioned above. In this example, the administration of peptide in any of these forms takes place on 3 occasions at times 0, 1 and 2 months.

In this example, treatment may be continued according to the indication of primary outcome measures. The primary outcome measures are a change in peptide-induced IL-10+ (increase) and IFN-γ+ (decrease) peptide-reactive cells detected by the cytokine ELISPOT assay or similar changes in IL-10+ and IFN-γ+ cells reactive with epitopes of preproinsulin, IA-2 that had not been administered (ie so-called bystander effects). Further primary outcome measures will be changes in basal and stimulated C-peptide levels at 3, 6, and 12 months after commencing treatment and changes in insulin dosage and HbA1c versus placebo, each of which represent enhancement of endogenous insulin production. Any such favourable outcome measures will dictate cessation of therapy; conversely, continuation of presence of or reappearance of, for example IFN-γ+ cells recognising the therapeutic peptides, will dictate continuation of therapy.

In this example, subjects for the therapy are individuals identified as being at-risk of diabetes development in the next 5-10 years through the presence of circulating autoantibodies. Autoantibodies used for this identification are those against preproinsulin, IA-2 and GAD65 and also an autoantibody termed islet cell antibody (ICA). All subjects will have at least one high risk HLA molecule, for example HLA-DR4, -DR3, -DQ8, -DQ2. Subjects can also be newly-diagnosed subjects with Type 1 diabetes, within 3 months of diagnosis and at least one circulating autoantibody as specified above.

### (2) A tolerance assay to monitor therapy for Type 1 diabetes.

Also, in accordance with a further aspect of the present invention, we describe hereinafter a tolerance assay that is made up of our peptides plus a cytokine ELISPOT bioassay for use in the monitoring of intervention therapies in patients with, or at risk of Type 1 diabetes. Our identification of specific peptides and combinations of peptides has led to the solution of previous therapeutic problems , in that the peptides can be used (a) to reveal the presence of pathogenic CD4+ T lymphocytes in patients and (b) to reveal the presence of non-pathogenic suppressor CD4+ T lymphocytes that have been induced by preventive therapies. An important contribution is our demonstration that our highly preferred combination of 4 epitopes from 2 autoantigens is vastly superior in terms of its coverage of pathogenic and suppressive CD4+ T lymphocyte responses than any other peptides previously proposed. Thus our diagnostic approach is multi-epitope, multi-antigen screening to monitor the balance of pathogenic versus protective immune responses in patients undergoing therapeutic interventions for Type 1 diabetes.

An example of this approach is as follows. Peptides representing the epitopes having the sequences identified hereinbefore are synthesized by standard Fmoc chemistry to LMP grade and used singly or pooled into cocktails representing the best possible combined efficacies. In this example, a particular immune modulating treatment is commenced with the aim of halting or preventing the autoimmune processes that lead to Type 1 diabetes. An example of this intervention is a course of treatment with peptide immunotherapy, or the non-depleting monoclonal anti-CD3 antibody hOKT3 directed against T cells or an immune suppressive drug such as rapamycin. These therapies are administered for a defined period and then surrogate markers are measured in a tolerance assay to assess the effect of the therapy on pathogenic autoimmunity. An example of a surrogate marker to be used in this way is the cytokine ELISPOT detecting pathogenic (IFN-γ) and suppressor (IL-10) CD4+ T lymphocyte responses to single or cocktails of peptides identified as described above. Reduction or disappearance of pathogenic CD4+ T lymphocytes, or induction of suppressor CD4+ T lymphocytes would lead to a reduction or cessation of therapy. No change or a worsening of these surrogate markers would lead to continuation of therapy and/or the introduction of new reagents.

A further aspect of the invention therefore comprises a method of measuring the state of immunological tolerance of a patient to beta cells or cells involved in other autoimmune disease which comprises the following steps:-
(a) Extracting the patient's peripheral blood mononuclear cells
(b) Culturing these cells with any of the peptides or peptide combinations defined hereinbefore, or derived by the methodology described hereinbefore
(c) Applying a cytokine ELISPOT analysis to the cultured cells in order to quantitate the cellular production of cytokines eg interferon-γ and interleukin-10. The patients immunological tolerance to the cells is demonstrated by the presence of an increased number of interleukin-10 producing cells and a reduced number of interferon-γ producing cells.

### SEQUENCE LISTING

110> King's College London
   <110> PEAKMAN, MARK
<120> PEPTIDES for treatment of autoimmune diseases
<130> IT/ED/N16513
<150> 10/783,095
   <151> 2004-02-23
<150> GB 0402129.1
   <151> 2004-01-30
<150> GB 0404199.2
   <151> 2004-02-25
<160> 17
<170> PatentIn Ver. 3.2
<210> 1
   <211> 22
   <212> PRT
   <213> Artificial Sequence
<220>
   <223> Description of Artificial Sequence: Synthetic peptide
<400> 1
   Thr Pro Lys Thr Arg Arg Glu Ala Glu Asp Leu Gln Val Gly Gln Val
    1               5                  10                  15
   Glu Leu Gly Gly Gly Pro
               20
<210> 2
   <211> 24
   <212> PRT
   <213> Artificial Sequence
<220>
   <223> Description of Artificial Sequence: Synthetic peptide
<400> 2
   Glu Asp Leu Gln Val Gly Gln Val Glu Leu Gly Gly Gly Pro Gly Ala
   1 5 10 15
   Gly Ser Leu Gln Pro Leu Ala Leu
   20
<210> 3
   <211> 24
   <212> PRT
   <213> Artificial Sequence
<220>
   <223> Description of Artificial Sequence: Synthetic peptide
<400> 3
   Asp Leu Gln Val Gly Gln Val Glu Leu Gly Gly Gly Pro Gly Ala Gly
   1 5 10 15
   Ser Leu Gln Pro Leu Ala Leu Glu
   20
<210> 4
   <211> 20
   <212> PRT
   <213> Artificial Sequence
<220>
   <223> Description of Artificial Sequence: Synthetic peptide
<400> 4
   Gly Gly Gly Pro Gly Ala Gly Ser Leu Gln Pro Leu Ala Leu Glu Gly
   1 5 10 15
   Ser Leu Gln Lys
   20
<210> 5
   <211> 18
   <212> PRT
   <213> Artificial Sequence
<220>
   <223> Description of Artificial Sequence: Synthetic peptide
<400> 5
   Gly Ser Leu Gln Pro Leu Ala Leu Glu Gly Ser Leu Gln Lys Arg Gly
   1 5 10 15
   Ile Val
<210> 6
   <211> 17
   <212> PRT
   <213> Artificial Sequence
<220>
   <223> Description of Artificial Sequence: Synthetic peptide
<400> 6
   Gln Pro Leu Ala Leu Glu Gly Ser Leu Gln Lys Arg Gly Ile Val Glu
    1               5                  10                  15
   Gln
<210> 7
   <211> 21
   <212> PRT
   <213> Artificial Sequence
<220>
   <223> Description of Artificial Sequence: Synthetic peptide
<400> 7
   Ala Leu Glu Gly Ser Leu Gln Lys Arg Gly Ile Val Glu Gln Cys Cys
    1               5                  10                  15
   Thr Ser Ile Cys Ser
               20
<210> 8
   <211> 86
   <212> PRT
   <213> Artificial Sequence
<220>
   <223> Description of Artificial Sequence: Synthetic proinsulin peptide
<400> 8
   Phe Val Asn Gln His Leu Cys Gly Ser His Leu Val Glu Ala Leu Tyr
    1               5                  10                  15
   Leu Val Cys Gly Glu Arg Gly Phe Phe Tyr Thr Pro Lys Thr Arg Arg
               20                  25                  30
   Glu Ala Glu Asp Leu Gln Val Gly Gln Val Glu Leu Gly Gly Gly Pro
           35                  40                  45
   Gly Ala Gly Ser Leu Gln Pro Leu Ala Leu Glu Gly Ser Leu Gln Lys
       50                  55                  60
   Arg Gly Ile Val Glu Gln Cys Cys Thr Ser Ile Cys Ser Leu Tyr Gln
   65                  70                  75                  80
   Leu Glu Asn Tyr Cys Asn
                   85
<210> 9
   <211> 11
   <212> PRT
   <213> Artificial Sequence
<220>
   <223> Description of Artificial Sequence: Synthetic peptide
<400> 9
   Gln Pro Leu Ala Leu Glu Gly Ser Leu Gln Lys
   1               5                   10
<210> 10
   <211> 26
   <212> PRT
   <213> Artificial Sequence
<220>
   <223> Description of Artificial Sequence: Consensus peptide
<400> 10
   Gly Gly Gly Pro Gly Ala Gly Ser Leu Gln Pro Leu Ala Leu Glu Gly
   1               5                   10                  15
   Ser Leu Gln Lys Arg Gly Ile Val Glu Gln
               20                  25
<210> 11
   <211> 28
   <212> PRT
   <213> Artificial Sequence
<220>
   <223> Description of Artificial Sequence: Synthetic peptide
<400> 11
   Leu Ala Lys Glu Trp Gln Ala Leu Cys Ala Tyr Gln Ala Glu Pro Asn
   1               5                   10                  15
   Thr Cys Ala Thr Ala Gln Gly Glu Gly Asn Ile Lys
               20                  25
<210> 12
   <211> 24
   <212> PRT
   <213> Artificial Sequence
<220>
   <223> Description of Artificial Sequence: Synthetic peptide
<400> 12
   Lys Leu Lys Val Glu Ser Ser Pro Ser Arg Ser Asp Tyr Ile Asn Ala
   1               5                   10                  15
   Ser Pro Ile Ile Glu His Asp Pro
               20
<210> 13
   <211> 20
   <212> PRT
   <213> Artificial Sequence
<220>
   <223> Description of Artificial Sequence: Synthetic peptide
<400> 13
   Ser Phe Tyr Leu Lys Asn Val Gln Thr Gln Glu Thr Arg Thr Leu Thr
   1               5                   10                  15
   Gln Phe His Phe
               20
<210> 14
   <211> 20
   <212> PRT
   <213> Artificial Sequence
<220>
   <223> Description of Artificial Sequence: Synthetic peptide
<400> 14
   Pro Lys Pro Pro Lys Pro Val Ser Lys Met Arg Met Ala Thr Pro Leu
   1               5                   10                  15
   Leu Met Gln Ala
               20
<210> 15
   <211> 21
   <212> PRT
   <213> Artificial Sequence
<220>
   <223> Description of Artificial Sequence: Synthetic peptide
<400> 15
   Leu Leu Glu Ser Gln Ile Ala Thr Ile Glu Gln Ser Ala Pro Ser Gln
   1               5                   10                  15
   Ser Asp Gln Glu Gln
               20
<210> 16
   <211> 22
   <212> PRT
   <213> Artificial Sequence
<220>
   <223> Description of Artificial Sequence: Synthetic peptide
<400> 16
   Ala Gly Lys Ser Val Ala Thr Asn Leu Ile Gly Arg Ser Leu Ala Glu
    1               5                  10                  15
   Lys Leu Asn Ser Ser Val
               20
<210> 17
   <211> 23
   <212> PRT
   <213> Artificial Sequence
<220>
   <223> Description of Artificial Sequence: Synthetic peptide
<400> 17
   Cys Gln Met Val Ser Ser Val Asp Phe Val Pro Pro Met Ala Ala Leu
    1               5                  10                  15
   Glu Glu Lys Gly Ile Leu Phe
               20

## Claims

1. A peptide consisting of the sequence GGGPGAGSLQPLALEGSLQKRGIVEQ (SEQ ID NO: 10), or a fragment thereof, said fragment consisting of GGGPGAGSLQPLALEGSLQK (SEQ ID NO: 4), GSLQPLALEGSLQKRGIV (SEQ ID NO: 5), QPLALEGSLQKRGIVEQ (SEQ ID NO: 6), or QPLALEGSLQK (SEQ ID NO: 9).

2. A composition comprising a first peptide as claimed in claim 1, said composition further comprising a peptide or peptides having a sequence or sequences selected from the group consisting of
a. IA-2 752-75 (SEQ ID NO: 12);
b. IA-2 853-72 (SEQ ID NO: 13);
c. IA-2 709-36 (SEQ ID NO: 11);
d. IA-2 752-75 (SEQ ID NO: 12) and IA-2 853-72 (SEQ ID NO: 13);
e. IA-2 709-36 (SEQ ID NO: 11) and IA-2 752-75 (SEQ ID NO: 12);
f. IA-2 709-36 (SEQ ID NO: 11) and IA-2 853-72 (SEQ ID NO: 13); and
g. IA-2 709-36 (SEQ ID NO: 11) and IA-2 752-75 (SEQ ID NO: 12) and IA-2 853-72 (SEQ ID NO: 13).

3. A composition according to claim 2, for use in the therapeutic or preventive treatment of Type 1 diabetes mellitus (T1DM).

4. A peptide or composition according to any preceding claim, in which the peptide or each peptide is conjugated or otherwise combined with a tolerance-promoting adjuvant or tolerance promoting cells.

5. A peptide or composition according to any preceding claim, in which the peptide or each peptide is conjugated or otherwise combined with dendritic cells or other antigen presenting cells.

6. A peptide or composition according to any one of claims 1 to 5 which is suitable for administration by parenteral or oral topical routes, including intradermal, subcutaneous or intravenous injection, or nasally or epicutaneously.

7. A peptide according to claim 1 for use in the therapy or prophylaxis of Type 1 diabetes mellitus (T1DM).

8. A method of measuring the state of immunological tolerance of a patient to beta cells or other cells affected or implicated in Type 1 diabetes mellitus, which comprises the following steps:
a. Incubating the patient's peripheral blood mononuclear cells *in vitro* with any of the peptide or compositions as defined in any one of claims 1 to 5; and
b. Applying a cytokine ELISPOT analysis to the incubated cells in order to quantitate the cellular production of interferon-γ and interleukin-10, wherein the patient's immunological tolerance to beta cells is demonstrated by the presence of an increased number of interleukin-10 producing cells and a reduced number of interferon-γ producing cells, compared to those present prior to treatment.

9. The use of any peptide or composition according to any one of claims 1 to 5, for the manufacture of a medicament for the therapy or prevention of Type 1 diabetes.

10. A method of assessing the potential of a peptide for use in the therapy or prevention of Type 1 diabetes, which comprise subjecting a candidate peptide to a first ELISPOT assay for IFN-γ indicative of a pathogenic T cell response in blood, wherein, in the case of a positive response to the first assay, the candidate peptide is subjected to a second ELISPOT assay for IL-10 indicative of a regulatory T cell response to the peptide.

## Patentansprüche

1. Peptid bestehend aus der Sequenz GGGPGAGSLQPLALEGSLQKRGIVEQ (SEQ ID NO: 10), oder ein Fragment davon, das Fragment bestehend aus
GGGPGAGSLQPLALEGSLQK (SEQ ID NO: 4),
GSLQPLALEGSLQKRGIV (SEQ ID NO: 5),
QPLALEGSLQKRGIVEQ (SEQ ID NO: 6), oder
QPLALEGSLQK (SEQ ID NO: 9).

2. Zusammensetzung umfassend ein erstes Peptid gemäß Anspruch 1, die Zusammensetzung umfasst weiter ein Peptid oder Peptide mit einer Sequenz oder Sequenzen ausgewählt aus der Gruppe bestehend aus
a. IA-2 752-75 (SEQ ID NO: 12);
b. IA-2 853-72 (SEQ ID NO: 13);
c. IA-2 709-36 (SEQ ID NO: 11);
d. IA-2 752-75 (SEQ ID NO: 12) und IA-2 853-72 (SEQ ID NO: 13);
e. IA-2 709-36 (SEQ ID NO: 11) und IA-2 752-75 (SEQ ID NO: 12);
f. IA-2 709-36 (SEQ ID NO: 11) und IA-2 853-72 (SEQ ID NO: 13); und
g. IA-2 709-36 (SEQ ID NO: 11) und IA-2 752-75 (SEQ ID NO: 12) und IA-2 853-72 (SEQ ID NO: 13).

3. Zusammensetzung gemäß Anspruch 2 zur Verwendung in der therapeutischen oder vorbeugenden Behandlung von Typ 1 Diabetes mellitus (T1 DM).

4. Peptid oder Zusammensetzung gemäß einem der vorhergehenden Ansprüche, in welcher das Peptid oder jedes Peptid mit einem Toleranz fördernden Adjuvans oder Toleranz fördernden Zellen konjugiert oder anderweitig kombiniert ist.

5. Peptid oder Zusammensetzung gemäß einem der vorhergehenden Ansprüche, in welcher das Peptid oder jedes Peptid mit dendritischen Zellen oder anderen antigenpräsentierenden Zellen konjugiert oder anderweitig kombiniert ist.

6. Peptid oder Zusammensetzung gemäß einem der Ansprüche 1 bis 5, welche für eine Verabreichung über parenterale oder orale topische Wege, einschließlich intrakutaner, subkutaner oder intravenöser Injektion, oder nasal oder epikutan, geeignet ist.

7. Peptid gemäß Anspruch 1 zur Verwendung in der Therapie oder Vorbeugung von Typ 1 Diabetes mellitus (T1 DM).

8. Verfahren zum Messen des Status der immunologischen Toleranz eines Patienten gegenüber Betazellen oder anderen Zellen, die in Typ 1 Diabetes mellitus betroffen oder verwickelt sind, welches die folgenden Schritte umfasst:
a. Inkubieren der mononukleären Zellen des peripheren Blutes des Patienten *in vitro* mit einem der Peptide oder Zusammensetzungen wie in einem der Ansprüche 1 bis 5 definiert; und
b. Anwenden einer Zytokin ELISPOT Analyse auf die inkubierten Zellen, um die zelluläre Produktion von Interferon-γ und Interleukin-10 zu quantifizieren, wobei die immunologische Toleranz des Patienten gegenüber Betazellen durch die Anwesenheit einer erhöhten Anzahl von Interleukin-10 produzierenden Zellen und einer verringerten Anzahl von Interferon-γ produzierenden Zellen, verglichen zu denen, die vor der Behandlung anwesend waren, gezeigt wird.

9. Verwendung eines Peptid oder einer Zusammensetzung gemäß einem der Ansprüche 1 bis 5 zur Herstellung eines Medikaments zur Therapie oder Vorbeugung von Typ 1 Diabetes.

10. Verfahren zum Bewerten des Potenzials eines Peptids zur Verwendung in der Therapie oder Vorbeugung von Typ 1 Diabetes, welches Zuführen eines Kandidatenpeptids zu einem ersten ELISPOT Assay für IFN-γ, das auf eine pathogene T-Zell-Antwort im Blut schließen lässt, umfasst, wobei im Falle einer positiven Antwort auf den ersten Assay das Kandidatenpeptid zu einem zweiten ELISPOT Assay für IL-10, das auf eine regulatorische T-Zell-Antwort auf das Peptid schließen lässt, zugeführt wird.

## Revendications

1. Peptide consistant en la séquence GGGPGAGSLQPLALEGSLQKRGIVEQ (SEQ ID N° : 10) ou un fragment de celui-ci, ledit fragment consistant en GGGPGAGSLQPLALEGSLQK (SEQ ID N° : 4), GSLQPLALEGSLQKRGIV (SEQ ID N° : 5), QPLALEGSLQKRGIVEQ (SEQ ID N° : 6) ou QPLALEGSLQK (SEQ ID N° : 9).

2. Composition comprenant un premier peptide selon la revendication 1, ladite composition comprenant en outre un peptide ou des peptides ayant une séquence ou des séquences choisies dans le groupe comprenant
a. IA-2 752-75 (SEQ ID N° : 12) ;
b. IA-2 853-72 (SEQ ID N° : 13) ;
c. IA-2 709-36 (SEQ ID N° : 11) ;
d. IA-2 752-75 (SEQ ID N° : 12) et IA-2 853-72 (SEQ ID N° : 13) ;
e. IA-2 709-36 (SEQ ID N° : 11) et IA-2 752-75 (SEQ ID N° : 12) ;
f. IA-2 709-36 (SEQ ID N° : 11) et IA-2 853-72 (SEQ ID N° : 13) ; et
g. IA-2 709-36 (SEQ ID N° : 11) et IA-2 752-75 (SEQ ID N° : 12) et IA-2 853-72 (SEQ ID N° : 13).

3. Composition selon la revendication 2, pour son utilisation dans le traitement thérapeutique ou préventif du diabète sucré de type 1 (T1DM).

4. Peptide ou composition selon l'une quelconque des revendications précédentes, le peptide ou chaque peptide étant conjugué ou associé d'une autre façon à un adjuvant favorisant la tolérance ou à des cellules favorisant la tolérance.

5. Peptide ou composition selon l'une quelconque des revendications précédentes, dans lequel le peptide ou chaque peptide est conjugué ou combiné d'une autre façon à des cellules dendritiques ou d'autres cellules de présentation d'antigène.

6. Peptide ou composition selon l'une quelconque des revendications 1 à 5, qui est approprié(e) pour l'administration par voies parentérales ou topiques orales, comprenant l'injection intradermique, sous-cutanée ou intraveineuse, ou les voies nasale ou épicutanée.

7. Peptide selon la revendication 1, pour son utilisation dans la thérapie ou la prophylaxie du diabète sucré de type 1 (T1DM).

8. Procédé de mesure de l'état de tolérance immunologique d'un patient aux cellules bêta ou d'autres cellules affectées ou impliquées dans le diabète sucré de type 1, qui comprend les étapes suivantes :
a. incubation de cellules mononucléaires du sang périphérique du patient *in vitro*, avec l'un quelconque parmi les peptides ou compositions tels que définis dans l'une quelconque des revendications 1 à 5 ; et
b. application d'une analyse ELISPOT de cytokine aux cellules incubées afin de quantifier la production cellulaire d'interféron γ et d'interleukine 10, la tolérance immunologique du patient aux cellules bêta étant démontrée par la présence d'un nombre accru de cellules produisant l'interleukine 10 et un nombre réduit de cellules produisant l'interféron γ par rapport à celles présentes avant le traitement.

9. Utilisation d'un peptide ou d'une composition selon l'une quelconque des revendications 1 à 5, pour la production d'un médicament pour la thérapie ou la prévention du diabète de type 1.

10. Procédé d'évaluation du potentiel d'un peptide pour son utilisation dans la thérapie ou la prévention du diabète de type 1, qui comprend la soumission d'un peptide candidat à un premier essai ELISPOT d'IFN-γ indicatif d'une réponse en lymphocytes T pathogènes dans le sang, dans lequel, dans le cas d'une réponse positive au premier essai, le peptide candidat est soumis à un deuxième essai ELISPOT d'IL-10 indicatif d'une réponse en lymphocytes T régulateurs au peptide.
